# NEW EUROPEAN PATENT SPECIFICATION

(11) **EP 0 444 186 B2**
(45) Date of publication and mention of the opposition decision: **29.10.2003**
(45) Mention of the grant of the patent: 15.11.1995
(21) Application number: 90914564.1
(22) Date of filing: 14.09.1990
(51) Int. Cl.: A61K 39/39, A61K 35/74

(54) **PHARMACEUTICAL COMPOSITION THAT MAKES CELLS EXPRESSING MHC CLASS II ANTIGENS TARGETS FOR CYTOTOXIC T-CELLS**
PHARMAZEUTISCHE ZUSAMMENSETZUNG DIE ZELLEN, WELCHE MHC-KLASSE-II-ANTIGENE EXPRIMIEREN, ZU ZIELZELLEN FÜR CYTOTOXISCHE T-ZELLEN MACHT
COMPOSITION PHARMACEUTIQUE FAISANT DE CELLULES EXPRIMANT DES ANTIGENES DE LA CLASSE II DU COMPLEXE MAJEUR D'HISTOCOMPATIBILITE, DES CIBLES POUR LES LYMPHOCYTES T CYTOTOXIQUES

(30) Priority: 20.09.1989 SE 8903100
(43) Date of publication of application: 04.09.1991
(73) Proprietor: Pharmacia Aktiebolag, 112 87 Stockholm (SE)
(72) Inventor: KALLAND, Terje, S-240 21 Löddeköpinge (SE); HEDLUNG, Gunnar, S-223 51 Lund (SE); DOHLSTEN, Mikael, S-223 53 Lund (SE); LANDO, Peter, S-214 34 Malmö (SE)
(74) Representative: Woods, Geoffrey Corlett
(86) International application number: SE9000592
(87) International publication number: WO91004053

(56) References cited:
- EP-A- 0 223 579
- WO-A-91/10680
- SHCHEGLOVITOVA et al. (1987), Exp. Onkol. (USSR) Vol. 9 (1), 28-30
- SHCHEGLOVITOVA et al. (1989), Exp. Onkol. (USSR) Vol. 11(1), 73-74
- SHCHEGLOVITOVA et al. (1989), Exp. Onkol. (USSR) Vol. 11(2), 54-57
- NATURE, vol. 339, May 1989; J.D. FRASER, pp. 221-223
- DIALOG INFORMATION SERVICES, File 154: Medline 83-90, NLM accession no. 89235137; P.M. STUART et al.
- DIALOG INFORMATION SERVICES, File 154: Medline 83-90, NLM accession no. 89078460; M. MATTHES et al

## Description

The present invention concerns a new agent and a new method for the activation of T cells. Specifically the invention concerns a new agent and method for treating certain cancer forms and autoimmune processes.

### Background of the invention

MHC antigens or transplantation antigens, which in man are also called HLA antigens, play an essential part in T cell activation and the con-comitant immune response. Thus e.g. protein antigens are dependent on interactions with MHC antigens for presentation to and activation of T cells during a normal immune response. Likewise, MHC dependent presentation of autoantigens are of key importance in T cell dependent autoimmune processes.

A certain type of MHC antigens, i.e. the so called MHC class II antigens are expressed by a variety of normal cells either constitutionally or after activation (1). Among tumour cells, malignancies of the haematopoietic system commonly express MHC class II molecules. Although MHC class II antigens are not restricted to tumour cells, they may constitute an attractive target for therapy since most normal cells would be spared.

A potent agent capable of eliminating MHC class II antigen expressing cells may therefore be useful both for interruption of autoimmune processes and for the destruction of certain malignant cells.

### Summary of invention

Accordingly, the present invention concerns a method of treating malignancies involving cells expressing MHC class II antigens, said method comprising administering to a living body a composition including a substance making said cells targets for cytotoxic T cells, wherein the substance is staphylococcal enterotoxin.

The invention also comprises a pharmaceutical composition for treating these malignancies. The composition includes a substance making said cells targets for cytotoxic T cells, wherein the substance is staphylococcal enterotoxin.

The staphylococcal enterotoxin should have the ability of activating the cytotoxic T cells, redirect them to MHC class II antigens irrespective of nominal specificity and thereby inactivate or lyse the MHC class II expressing cells.

According to the invention it has been found that a preferred staphylococcal enterotoxin has the ability to direct T cells to lyse the target MHC class II antigen expressing cell. It is believed that the staphylococcal enterotoxin should interact with parts of the T cell receptor complex (TCR) and, more specifically, particular sequences in the T cell receptor V beta chains. Preferably the staphylococcal enterotoxin is selected from the group comprising of staphylococcal enterotoxines, SE:s, such as SEA. SEB, SEC, SEC1, SEC2, SEC3, SED or SEE, toxoids active fragments or peptides of these enterotoxins and substances having essentially the same mode of action, to interact with certain TCR V-beta sequences. Genetically or chemically modified substances based on the above mentioned structures could also be used. See also Fraser, Nature 339 (1989) 221-223 and Matthes et al., Eur J. Immunol 18 (1988) 1733-1737.

An example of a substance which does not fall within the scope of the invention is protein A. This protein emanates from Staphyloccccus but it is both structurally and functionally different from the staphylococcal enterotoxins.

Staphylococcus enterotoxins (SE), which are produced by Grampositive bacteria are a family of molecules of protein structure, that are commonly known for causing food poisoning (2). They are also potent T cell mitogens causing T cell activation and concomitant production of lymphokines (3-5). However, SE unlike most mitogens are strictly dependent on the presence of accessory cells expressing MHC class II molecules (6,7).

The substance used as active entity may comprise a combination of two or more enterotoxins, toxoids, active fragments or peptides thereof.

Moreover, strong circumstantial evidence has been presented that SEA directly interacts with certain T cell receptor families during their activation of lymphocytes (8,9).

Animal studies relating to the production of tumour killing substances and combatting of implanted tumors have been described by Shcheglovitova et al., Exp Onkul : (USSR) 9 (1987) 28-30, 11 (1989) 73-74 and 11 (1989) 54-57, No reference to MHC class II antigens has been made.

It has been previously suggested (WO-A1-89/09619) to use SEA for the treatment of cancer. According to this publication, however, SEA is used for the treatment of blood in vitro (= outside the patients body), and the SEA is removed before the activated cells are reinfused to the patient, whereas the discovery on which the present invention is based discloses that SEA and functionally similar agents can act as bifunctional crosslinkers in vitro. Activating and bringing T cells to tumour cells in the body can be of potential use for the in vivo treatment of certain types of cancer and autoimmune diseases.

The agent according to the present invention is preferably in the form of a pharmaceutical composition comprising the substance, together with a pharmaceutically acceptable vehicle or carrier.

The composition is furthermore preferably administered parenterally, and should thus include those adjuvants which are commonly used within this field. Thus the enterotoxin or fragment thereof could be administered in the form of lipid emulsions, or combined with human serum albumin or mixtures of amino acids The amount of the active component of the composition can vary within broad ranges e.g. 1 ng - 100 mg.

In addition to systemic administration it is also possible to use the composition according to the invention for local administration. The composition could e.g. be locally administered in an arthritic joint.

Conditions which may be treated by the compositions according to the invention are variuous forms of autoimmune diseases and tumor diseases e.g. lymphomas and leukemias.

The invention is further illustrated by the following examples which should not be construed as limiting the invention.

### Examples

To determine Staphylococcal enterotoxin directed cell mediated cytotoxicity (SDCC) we employed a panel of human anti-HLA-A2 cytotoxic T cell lines as effector cells and the HLA-A2⁻DR⁺ EBV transformed RAJI B-cell line, the R.2.2 HLA-DR⁻ mutant of the RAJI cell line and the HLA-A2⁺DR⁺ EBV transformed BSM B-cell line. The anti-HLA-A2 specific T cell lines were established from primary MLC cultures and repeated restimulations with mitomycin-treated HLA-A2⁺ stimulator cells and recombinant IL-2 (20 units/ml). These T cell lines strongly lysed the specific HLA-A2 expressing target but not irrelevant targets. The effect of Staphyloccal enterotoxin (SE) A, B, C1 and D was studied using SE from Tox Tech (Madison, WI., USA). To demonstrate that HLA-DR is the target molecule in SDCC we used the above described panel of target cells and monoclonal antibodies (mAb) directed to HLA-DR, MHC Class II, W6/32 and CD 23.

### Chromium labeling and incubation of the target cells with SEA:

0.75x10⁶ target cells and 150 µCi ⁵¹chromium (Amersham Corp., Arlington Hights, England) were incubated for 45 minutes at 37°C in a volume of 100 µl. The cells were kept in complete medium containing RPMI-1640 medium (Gibco, Paisley, GBR) supplemented with 2.8 % (v/v) 7,5 % NaHCO₃, 1 % sodium pyrovate, 2 % 200 mM L-glutamine, 1 % 1M Hepes, 1 % 10mg/ml gentamicin and 10 % fetal calf serum (FCS, Gibco, Paisley, GBR). After the incubation the cells were washed once in complete medium without FCS and incubated 60 minutes at 37°C and washed and resuspended in complete medium containing 10 % FCS: 5x10³ target cells were added to each well of U-bottom 96-well microtiter plates (Costar, Cambridge, USA).

### Cytotoxicity assay

The effector cells were added to the wells at various effector/target cell ratios. The final volume in each well was 200 µl. Each test was done in triplicate. The plates were incubated 4 hours at 37°C after which the released chromium was harvested using SCS Harvesting frames (Skatron, Norway). The amount ⁵¹Cr was determined in a gamma-counter (Cobra Auto-gamma, Packard). The percentage cytotoxicity was computed by the formula %cytotoxicity = (X-M)/(T-M) * 100, were X is the chromium release as cpm obtained in the test sample, M is the spontaneous chromium release of target cells incubated with medium, and T is the total chromium release obtained by incubating the target cells with 1% sodium dodecyl sulfate.

### Results

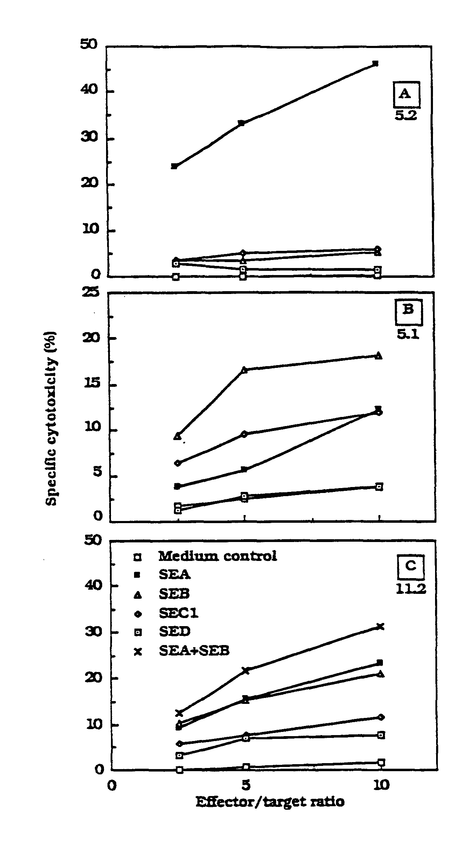

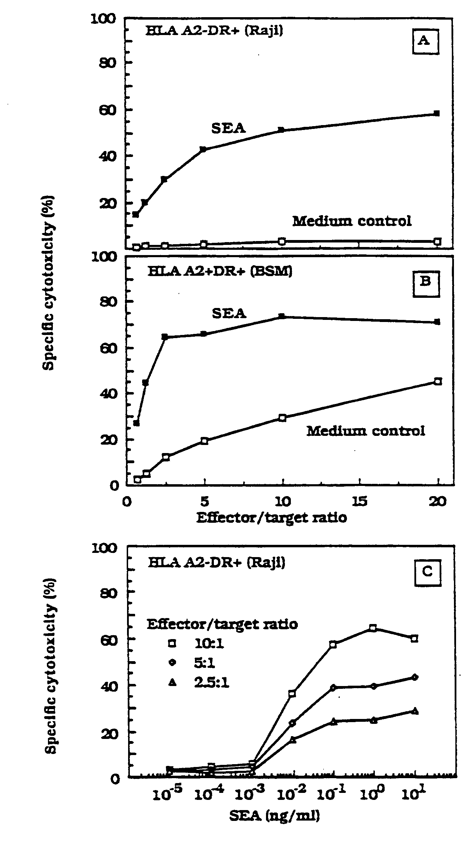

Human allospecific T cell lines, which demonstrated exquisite specificity for HLA-A2 showed strong cytotoxicity against the irrelevant HLA-A2-DR⁺ RAJI target cell and increased cytotoxicity against the specific HLA-A2⁻DR⁺ BSM target cell, when SEA was added in the assay (Fig. 1 A-B). The T cell mediated SE directed cellular cytotoxicity (SDCC) occured at very low concentrations of SE, with maximal effect at 10-0.1 ng/ml and half maximal effect was seen at about 0.01 ng/ml (Fig. 1 C). The SDCC phenomenon could be induced by several of the SE. The 5.2 T cell line showed selective reactivity towards SEA, whereas the 5.1 and 11.2 lines reacted with several SE (Fig. 2 A-C). Activation of T cells by the combination of SEA and SEB resulted in an additive increase in cytotoxicity to the RAJI target cell (Fig. 2 C). Blocking studies with monoclonal antibodies (mAb) directed to different cell surface structures demonstrated that the mAb G8, which has recently been shown to interact with a SEA binding site on the HLA-DR molecule, strongly inhibited the SDCC (Table 1. Expt #1). In contrast, the HB 96 mAb, which interacts with a monomorphic MHC Class II determinant, unrelated to the SEA binding epitope and the MHC Class I and CD23 mAb did not show any inhibitory activity (Table 1. Expt.#1). Preincubation experiments with SE demonstrated that the binding of SE to the RAJI cells and not to the T cell line was a prerequisite for the induction of SDCC against the RAJI cells (Table 1. Expt.#2). The MHC Class II negative RJ2.2.5 mutant of the RAJI cell line was found to be completely resistent to SDCC, while the Class II expressing parental cell line was an excellent target (Table 1. Expt.#3). These observerations implicates that HLA-DR is the main target molecule in SDCC.

Legend to Figure 1. SEA induced T cell mediated cytotoxicity against RAJI and BSM target cells. The cytotoxicity of an anti-HLA-A2 allospecific T cell line to the RAJI (HLA-A2⁻DR⁺) and BSM (HLA-A2⁺DR⁺) lymphoblastoid cell lines in the presence or absence of 1 ng/ml SEA was examined at various effector/target ratios in a standard 4 hour ⁵¹-chromium release assay and is expressed as % specific cytotoxicity. Figure 1 C demonstrates the dose-response of SEA at concentrations 10⁻⁵ to 10² ng/ml. SEA was added directly into the microtiter wells together with ⁵¹-Chromium label led RAJI target cells and effector T cells at indicated effector/target cell ratios. Addition of SEA to the target cells in the absence of effector cells did not result in any significant change in the spontaneous release of ⁵¹-chromium.

Legend to Figure 2. Induction of SDCC by SEA, SEB, SEC1 and SED. The cytotoxicity of the anti-HLA-A2 allospecific T cell lines 5.2, 5.1 and 11.2 against RAJI target cells in the presence or absence of the various SE at concentration 1 ng/ml was examined in a 4 hour ⁵¹-chromium release assay.

**Table 1.**

| HLA-DR is the target molecule in SDCC | | | | | | |
|---|---|---|---|---|---|---|
| | | Additive | | % Cytotox at E/T ratio^{A} | | |
| Effector | Target | SE | mAb | A | B | C |
| Expt.#1. | | | | | | |
| 5.2 | RAJI | - | - | 1 | 0 | 0 |
| | | + | - | 30 | 19 | 14 |
| | | + | HB96 | 31 | 23 | 16 |
| | | + | G8 | 8 | 6 | 3 |
| | | + | W6/32 | 37 | 28 | 19 |
| | | + | CD23 | 40 | 27 | 19 |
| Expt.#2. | | | | | | |
| 11.2 | RAJI | - | | 2 | 0 | 0 |
| | | + | | 56 | 34 | 14 |
| | RAJI/SE | - | | 33 | 28 | 12 |
| 11.2/SE | RAJI | - | | 2 | 0 | 0 |
| Expt.#3. | | | | | | |
| 12.1 | RAJI | - | | 3 | 2 | 3 |
| | | + | | 43 | 30 | 22 |
| | R12.2.5 | - | | 10 | 11 | 8 |
| | | + | | 12 | 9 | 6 |
| | RAJI/SE | - | | 32 | 24 | 21 |
| | R12.2.5/SE | - | | 12 | 9 | 7 |

| | | | | | | |
|---|---|---|---|---|---|---|
| ^{A} Cytotoxicity of anti-HLA-A2 allospecific T cell lines against HLA-DR⁺ RAJI cells and MHC Class II negative Raji mutant R.2.2.5 cells. The different mAb were added at the initiation of the culture at an optimal concentration of 20 ug/ml purified ascites Ig (HB 96 and W6/32) or at a ascites dilution 1/300 (G8 and CD23). SE was added to the assay at 0.1 ng/ml (Expt.#1.), 10 ng/ml (expt. #2.) or 1 ng/ml (Expt.#3.). Preincubation of target and effector cells was performed with SE at 1 ng/ml (Expt.#2: RAJI/SE and 11.2/SE) or 10 ng/ml (Expt. #3: RAJI/SE and R.2.2.5/SE) for 30 minutes at room temperature. The cells were extensiveley washed prior to use in the assay. Addition of SE to the different target cells did not result in any significant change in the spontaneous release of ⁵¹-chromium. The effector/target (E/T) ratios were 20:1, 10:1 and 5:1 in Expt.#1 and #3 and 30:1, 10:1 and 3:1 in Expt.#2. | | | | | | |

### Composition for injections

1 ng - 100 mg SEA (available from Tox Tech Madison, W1) is dissolved in 0,1 - 5 ml PBS (Phosphate Buffered Saline) and optionally human serum albumin or another conventionally used vehicle.

Injections for administration are intravenous bolus injections including 1-5 ml or continuous infusion of 500 ml during a period of 1-24 h.

### For demonstration of in vivo anti-tumor effects of SE the following two experimental approaches are performed:

1. 10⁶ murine MHC II⁺ A20 lymphoma cells in 0,3 ml Saline are injected subcutaneously in C57BI/6 mice. The mice are treated with 1 ug of SEA in 0,5 ml Saline intravenously or as control Saline alone. The treatment is given as one dose every week. Tumor growth is followed every day by measurement of total tumor volume. After 4 weeks of therapy the mice are sacrificed.
2. 10⁵ murine MHC II⁺ transfected B 16 melanoma cells in 0,3 ml Saline are injected intravenously in C57Bl/6 mice. The mice are treated with 1 ug of SEA in 0,5 ml Saline intravenously or as control Saline alone. The treatment is given as one dose every week. Mice are sacrificed after 3 and 4 weeks of therapy and the number of lung metastasis are evaluated.

### REFERENCES

1. Guillemot, F., Auffrey, C., Orr, H.T., and Strominger, J.: MHC antigen genes In: Molecular Immunology (Harnes, B.D. and Glover, D.M. (Eds.)) pp 81-143; IRL Press, Oxford, 1988.
2. Bergdoll, M.S.: Enterotoxins In: Staphylococci and Staphyloccocal Infections Vol. II (Easman, C.S.F. and Adlam, C. (Eds.)) pp 559-598; Acad. Press, New York, 1983.
3. Langford, M.P., Stanton, G.J., Johnson, H.M.: Biological effects of Staphylococcal enterotoxin A on human peripheral blood lymphocytes, Infect. Immun. 22:62-8, 1978.
4. Carlsson, R. Sjögren, H.O.: Kinetics of IL-2 and interferon-gamma production, expression of IL-2 receptors, and cell proliferation in human mononuclear cells exposed to staphylococcal enterotoxin A. Cell. Immunol. 96:175-181, 1985.
5. Fisher, H., Dohlsten, M., Andersson, U., Hedlund, G., Ericsson, P-E., Hansson, J., Sjögren, H.O.: Production of TNF-a and TNF-b by staphylococcal enterotoxin A activated human T cells. J. Immunol. Methods, in press, 1989.
6. Fisher, H., Dohlsten, M., Lindvall, M., Sjögren, H.O., Carlsson, R.: Binding of staphylococcal enterotoxin A to HLA-DR on B cell lines. J. Immunol. 142:3151-3157, 1989.
7. Mollick, J.A., Cook, R.G., Rich, R.R.: Class II MHC molecules are specific receptors for staphylococus enterotoxin A. Science 244:817-820, 1989.
8. Lando, P.A., Dohlsten, M., Kalland, T., Sjögren, H.O., Carlsson, R.: The TCR-CD3 complex is required for activation of human lymphocytes with staphylococcal enterotoxin A. Scand. J. Immunol., in press, 1989.
9. White, J., Herman, A., Pullen, A.M., Kubo, R., Kappler, J.W., Marrack, P.: The Vb-specific superantigen staphylococcal enterotoxin B: Stimulation of mature T cells and clonal deletion in neonatal mice. Cell 56:27-36, 1989.

## Claims

1. The use of a staphylococcal enterotoxin for the manufacture of a pharmaceutical composition for parenteral administration to a patient suffering from cancer involving cells expressing MHC class II antigen.

2. The use according to claim 1 wherein said staphylococcal enterotoxin is staphylococcal enterotoxin A.

## Patentansprüche

1. Verwendung eines Staphylokokkenenterotoxins zur Herstellung einer pharmazeutischen Zusammensetzung zur parenteralen Verabreichung an einen Patienten, der an Krebs leidet, bei welchem Zellen eine Rolle spielen, die MHC Klasse II Antigen exprimieren.

2. Verwendung gemäß Anspruch 1, worin das Staphylokokkenenterotoxin Staphylokokkenenterotoxin A ist.

## Revendications

1. Utilisation d'une entérotoxine staphylococcique pour la préparation d'une composition pharmaceutique destinée à l'administration parentérale à un patient souffrant d'un cancer impliquant des cellules exprimant un antigène de classe II du complexe majeur d'histocompatibilité (CMH).

2. Utilisation selon la revendication 1, dans laquelle ladite eritérotoxine staphylococcique est l'entérotoxine staphylococcique A.
